(19) Europäisches Patentamt
European Patent Office
Office européen des brevets

(11) Numéro de publication: **0 238 374 B1**

(12) **FASCICULE DE BREVET EUROPEEN**

(45) Date de publication de fascicule du brevet: **21.09.94**

(51) Int. Cl.[5]: **C07D 209/58**, C07D 209/66, C07D 209/76, C08K 5/34

(21) Numéro de dépôt: **87400293.4**

(22) Date de dépôt: **09.02.87**

(54) **Procédé de préparation d'imides halogénés, compositions les contenant et leur application comme agents d'ignifugation.**

(30) Priorité: **12.02.86 FR 8601917**

(43) Date de publication de la demande:
**23.09.87 Bulletin 87/39**

(45) Mention de la délivrance du brevet:
**21.09.94 Bulletin 94/38**

(84) Etats contractants désignés:
**AT BE CH DE ES FR GB GR IT LI LU NL SE**

(56) Documents cités:
**EP-A- 0 101 785**
**FR-A- 2 369 261**
**FR-A- 7 732 331**
**US-A- 3 705 127**
**US-A- 3 873 567**

**JP-74-045062**

**Industrial and Engineering Chemistry Product Research 8(4), 397-8 (1969)**

(73) Titulaire: **ELF ATOCHEM S.A.**
**4 & 8, Cours Michelet**
**La Défense 10**
**F-92800 Puteaux (FR)**

(72) Inventeur: **Bonnet, Evelyne**
**18 Grande Avenue**
**F-60260 Lamorlaye (FR)**
Inventeur: **Gagnieur, André**
**7 Route de la Nation**
**F-69270 Rochetaillee sur Saone (FR)**
Inventeur: **Gurtner, Bernard**
**5 Boulevard Agutte Sembat**
**F-38000 Grenoble (FR)**

**EP 0 238 374 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne la synthèse de polyhalogénoimides et, plus particulièrement, celle d'imides dérivés d'acides dicarboxyliques halogénés comme l'acide tétrabromophtalique.

Les polyhalogénoimides, notamment les tétrabromophtalimides et bis(tétrabromophtalimides), sont des composés bien connus qui trouvent une application comme retardateurs de flamme dans de nombreuses matières inflammables, en particulier dans les matières plastiques (voir, par exemple, l'article de S.M. SPATZ et al intitulé "Some N-substituted tetrabromophtalimide fire-retardant additives" dans Industrial and Engineering Chemistry Product Research and Development, vol. 8, n° 4 (1969) pages 397-398, ainsi que les brevets US 3 873 567 et FR 2 369 261 et les demandes JP 74-045062 et 75-064337).

Cependant, les procédés de préparation décrits dans les références précitées donnent des rendements médiocres et fournissent des produits le plus souvent colorés en jaune ou qui se colorent lors de leur mise en oeuvre et confèrent ainsi aux matières plastiques (compounds ou objets moulés) une coloration rédhibitoire et inacceptable dans bon nombre d'utilisations. D'autre part, ces produits contiennent très souvent des matières qui sont volatiles aux températures de mise en oeuvre dans certains matériaux polymériques et entraînent la corrosion des moules. En outre, l'emploi pour leur préparation de solvants organiques (notamment xylène, toluène, alcool, acide acétique), sélectionnés le plus souvent en raison de leur aptitude à former avec l'eau des mélanges azéotropiques permettant d'entraîner l'eau de condensation produite par la réaction d'imidification ou à dissoudre l'anhydride d'acide dicarboxylique halogéné, nécessite des opérations coûteuses de séparation et de récupération de ces solvants, ainsi que des moyens de séchage adaptés à l'élimination des vapeurs de solvants organiques.

Les inconvénients susmentionnés se rencontrent notamment dans le cas des polyhalogénoimides dérivés d'hydrazine et d'anhydrides d'acides dicarboxyliques halogénés. Il a maintenant été trouvé qu'il n'est pas indispensable d'utiliser un solvant organique pour dissoudre l'anhydride et/ou éliminer l'eau de condensation et qu'en opérant en milieu aqueux sous certaines conditions, on peut obtenir avec des rendements très élevés et sans problèmes de rejet d'effluents et d'environnement, des produits incolores ou très peu colorés qui, sans purification préalable, conviennent parfaitement à l'ignifugation des matières macromoléculaires, y compris celles dont la mise en oeuvre s'effectue à haute température, particulièrement au-dessus de 250 °C.

Le procédé selon l'invention qui consiste à condenser l'hydrazine avec un anhydride d'acide dicarboxylique halogéné, est caractérisé en ce que l'on effectue la réaction en milieu aqueux, à une température allant de 40 à 225 °C, avec un rapport molaire anhydride/hydrazine sensiblement égal à 2.

Parmi les anhydrides halogénés utilisables, on peut citer plus particulièrement :

- les anhydrides d'acides dicarboxyliques aromatiques (benzène, naphtalène, anthracène), notamment ceux de formule générale:

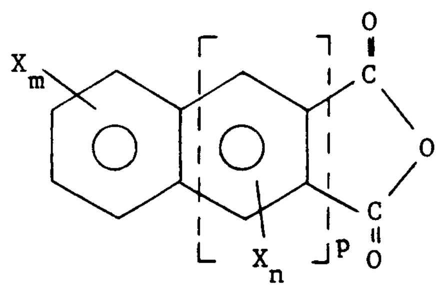

(I)

dans laquelle X représente un atome de brome ou de chlore, m est un nombre entier allant de 2 à 4, n et p sont des nombres entiers allant de 0 à 2 ;

- les anhydrides d'acides dicarboxyliques cycloaliphatiques, tels que l'acide hexabromo-1,4,5,6,7,7 bicyclo[2.2.1] heptène-5 dicarboxylique-2,3 et son homologue chloré (acide chlorendique), correspondant à la formule :

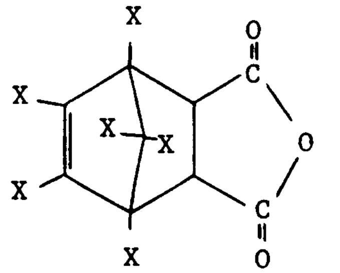

(II)

dans laquelle X a la même signification que ci-dessus. On utilise de préférence les anhydrides bromophtaliques, par exemple l'anhydride dibromo-3,4 (ou 3,6 ou 4,5) phtalique, l'anhydride tribromo-3,4,6 phtalique et plus particulièrement l'anhydride tétrabromophtalique.

Conformément à la présente invention, on peut utiliser un seul anhydride ou un mélange de deux ou plusieurs anhydrides. Les anhydrides commerciaux contenant souvent une petite quantité d'acides minéraux peuvent être utilisés tels quels sans purification préalable. Il a en effet été constaté qu'on obtient des résultats particulièrement bons lorsque la solution ou dispersion aqueuse d'anhydride mise en oeuvre est fortement acide (pH inférieur à 4).

La quantité d'eau constituant le milieu réactionnel du procédé selon l'invention peut varier dans de larges limites, la seule condition étant qu'elle soit suffisante pour assurer la dispersion convenable des réactifs et permettre une bonne agitation. Cette condition est généralement réalisée en utilisant une quantité d'eau telle que la teneur en matières solides du milieu réactionnel soit comprise entre environ 5 et 75 % en poids, de préférence entre 20 et 40 %.

La réaction peut être effectuée sous pression atmosphérique à une température allant de 40 à 100 ° C ou, à condition d'opérer sous pression, à une température plus élevée pouvant aller jusqu'à 225 ° C. De préférence, on opère entre 100 et 225 ° C ce qui correspond à des pressions comprises entre 1 et 25 bars environ.

L'hydrazine sous forme d'hydrate ou de sel d'hydrazinium (par exemple sulfate, hydrohalogénure, acétate) peut être utilisée telle quelle ou sous forme de solution aqueuse diluée. De préférence, on utilise l'hydrate d'hydrazine sous forme de solution aqueuse commerciale. Le procédé selon l'invention est avantageusement mis en oeuvre en introduisant progressivement l'hydrazine dans la solution ou dispersion d'anhydride halogéné préalablement chauffée et maintenue sous agitation.

La durée de réaction peut varier dans de larges limites, mais est généralement comprise entre 1 et 20 heures. Aprés refroidissement du milieu réactionnel, la suspension solide obtenue est filtrée et éventuellement lavée à l'eau jusqu'à neutralité, puis on séche le produit par les moyens de séchage classiques.

Lorsque le procédé selon l'invention est réalisé à une température au moins égale à 140 ° C, le produit obtenu est généralement constitué par le bis-imide répondant à la structure générale suivante:

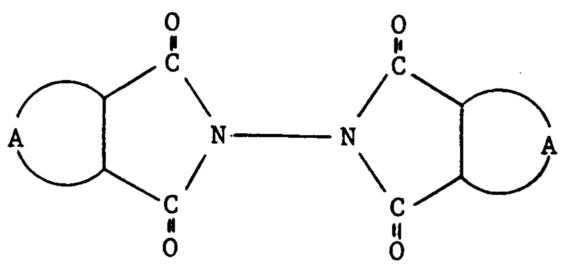

(III)

dans laquelle A représente le reste de l'anhydride halogéné mis en oeuvre.

Aux températures plus basses, le produit obtenu contient, en plus du bis-imide de formule (III), une proportion variable pouvant atteindre 80 % en poids d'un mélange sensiblement équimolaire de l'anhydride halogéné de départ et du N-amino-imide correspondant de structure générale :

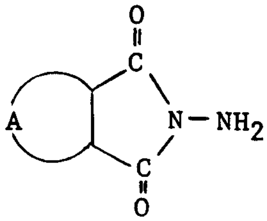

(IV)

où A a la même signification que précédemment, ce N-amino-imide pouvant être sous forme de sel (sulfate, hydrohalogénure, acétate). La teneur du produit obtenu en bis-imide (III) est en général d'autant plus grande que la température réactionnelle est proche de 140 ° C.

Qu'il s'agisse de bis-imides purs ou de mélanges bis-imide + N-amino-imide + anhydride, les produits obtenus conformément au procédé selon l'invention conviennent particulièrement bien comme retardateurs de flamme dans les matières plastiques de toute nature. Leur incorporation dans ces matières peut être effectuée par toute méthode connue à des doses allant de 5 à 40 % par rapport au poids de matière inflammable.

Les exemples suivants, dans lesquels les parties et les pourcentages sont exprimés en poids, illustrent l'invention sans la limiter.

EXEMPLE 1

Dans un réacteur en verre, muni d'un agitateur et d'un dispositif de reflux, on disperse dans 3000 parties d'eau distillée 1114 parties d'anhydride tétrabromophtalique commercial. Le pH de la suspension est de 2,2.

Aprés avoir porté cette suspension à 60 ° C, on ajoute progressivement en 20 à 30 minutes 60 parties d'hydrate d'hydrazine 100 %, puis on porte le mélange réactionnel à 100 ° C. Pendant les sept premières heures de réaction à cette température, la dispersion est jaune. On ajoute ensuite 1600 parties d'eau distillée et maintient à 100 ° C pendant encore 7 heures. On obtient alors une suspension blanche à blanc-ivoire qu'on refroidit et filtre sur büchner. Aprés lavage à l'eau jusqu'à pH neutre et séchage sous vide à 110-120 ° C, on obtient avec un rendement supérieur à 96 % un produit blanc-ivoire dont l'analyse montre qu'il contient environ :

40 % de N,N′-bis(tétrabromophtalimide)
30,5 % de N-amino-tétrabromophtalimide
29,5 % d'anhydride tétrabromophtalique.

Ces pourcentages ont été déterminés par extractions successives à l'aide de solvants spécifiques de l'anhydride tétrabromophtalique et du N-amino-tétrabromophtalimide (respectivement le méthanol et le diméthylsulfoxyde), le N,N′-bis(tétrabromophtalimide) étant insoluble dans ces solvants. D'autres méthodes d'identification (analyses spectroscopiques RMN et IR, analyses thermiques ATD et ATG) ont également été utilisées pour identifier les différents composés.

EXEMPLE 2

On opère comme à l'exemple 1, mais en ajoutant 2,5 parties d'acide sulfurique à 96 % à la suspension aqueuse d'anhydride tétrabromophtalique. Le pH est alors de 1,1.

On obtient avec un rendement supérieur à 98 % un produit blanc-ivoire contenant environ :

29 % de N,N′-bis(tétrabromophtalimide)
36 % de N-amin-tétrabromophtalimide (en partie sous forme de sulfate)
et
35 % d'anhydride tétrabromophtalique.

EXEMPLE 3

Dans un autoclave de un litre, muni d'un agitateur, on disperse 186 parties d'anhydride tétrabromophtalique commercial dans 600 parties d'eau distillée portée à 115 ° C à l'aide d'une enveloppe chauffante. Le pH de la suspension est de 2,2.

Au moyen d'une pompe, on introduit progressivement dans la suspension 10 parties d'hydrate d'hydrazine 100 % diluées avec 100 parties d'eau distillée. En début d'introduction, la pression est

d'environ 3 bars; en fin d'introduction, la pression est d'environ 5 bars et la température de 130 °C.

On maintient ces conditions (5 bars, 130 °C) pendant environ 8 heures, puis on refroidit le mélange réactionnel par circulation d'eau froide et on filtre la suspension obtenue. Aprés lavage à l'eau jusqu'à pH neutre et séchage sous vide à 100-110 °C, on obtient avec un rendement de 97 % un produit blanc contenant environ :

95 % de N,N′-bis(tétrabromophtalimide)

2,6 % de N-amino-tétrabromophtalimide

et

2,4 % d'anhydride tétrabromophtalique.

EXEMPLE 4

Dans un autoclave de 6 litres équipé d'un agitateur et d'un moyen de chauffage (par exemple, double enveloppe), on disperse 1335 parties d'anhydride tétrabromophtalique commercial dans 4500 parties d'eau distillée. Le pH de la suspension est de 2,2.

On porte cette suspension à 150 °C, puis on y introduit progressivement 72 parties d'hydrate d'hydrazine diluées dans 125 parties d'eau distillée et on maintient ensuite la pression entre 5 et 6 bars pendant environ 5 heures.

On refroidit ensuite le mélange réactionnel par circulation d'eau froide, puis on filtre la suspension. Aprés lavage à l'eau jusqu'à pH neutre et séchage sous vide à 100-110 °C, on obtient avec un rendement de 97 % un produit blanc constitué par le N,N′-bis(tétrabromophtalimide) pratiquement pur.

EXEMPLE 5

Dans le même appareillage qu'à l'exemple 1, on disperse dans 3000 parties d'eau distillée 890 parties d'anhydride chlorendique (anhydride de l'acide hexachloro-1,4,5,6,7,7 bicyclo [2.2.1] heptène-5 dicarboxylique-2,3), puis on porte le mélange à 60 °C. Le pH de la suspension est d'environ 2.

On ajoute ensuite progressivement (en 20 à 30 minutes) 60 parties d'hydrate d'hydrazine 100%, puis on porte la suspension à 100 °C et maintient à cette température pendant environ 12 heures.

Par distillation, on élimine une partie de l'eau du mélange réactionnel jusqu'à l'obtention d'une pâte humide qu'on essore, puis lave à l'eau froide jusqu'à neutralité et sèche à l'étuve sous vide à 100 °C pendant 8 heures.

On obtient ainsi un produit blanc-ivoire contenant environ :

90 % de N,N′-bis(chlorendimide)

5 % de N-amino-chlorendimide

et

5 % d'anhydride chlorendique.

EXEMPLE 6

Dans le même appareillage qu'à l'exemple 1, on disperse dans 10000 parties d'eau distillée 800 parties d'anhydride tétrachlorophtalique.

La suspension dont le pH est d'environ 2 est ensuite portée à 90 °C et on ajoute en environ 2 heures 70 parties d'hydrate d'hydrazine 100 %. Aprés environ 12 heures de réaction à 98 °C, on refroidit, filtre la suspension et lave à l'eau jusqu'à pH neutre.

Aprés séchage sous vide à l'étuve, on obtient avec un rendement supérieur à 96 % un produit blanc composé d'environ :

68 % de N,N′-bis(tétrachlorophtalimide)

16 % de N-amino tétrachlorophtalimide

et

16 % d'anhydride tétrachlorophtalique.

EXEMPLE 7

Dans le même appareillage qu'à l'exemple 1, on disperse dans 4000 parties d'eau distillée 372 parties d'anhydride tétrachlorophtalique et 603 parties d'anhydride tétrabromophtalique.

La suspension ainsi obtenue (pH 2 environ) est ensuite portée à 90 °C, puis on ajoute en 3 à 4 heures 65 parties d'hydrate d'hydrazine 100 % et laisse réagir pendant 12 heures à 98 °C.

Aprés filtration, lavage à l'eau jusqu'à pH neutre et séchage sous vide à 110-120 °C pendant 8 heures, on obtient avec un rendement supérieur à 96 % un produit blanc composé d'environ :

- 50 % d'un mélange de N,N′-bis (tétrachloro et bromophtalimides),
- 25 % d'un mélange de N-amino tétrachlorophtalimide et de N-amino tétrabromophtalimide, et
- 25 % d'un mélange d'anhydrides tétrachloro et tétrabromophtaliques.

EXEMPLE 8

Dans le même appareillage qu'à l'exemple 1, on disperse dans 500 parties d'eau distillée 153 parties d'anhydride hexabromo-1,4,5,6,7,7 bicyclo [2.2.1] heptène-5 dicarboxylique-2,3, ci-aprés désigné "anhydride bromendique" et préparé à partir d'hexabromocyclopentadiène et d'anhydride maléïque. Le pH de la suspension est d'environ 2,3.

On porte le mélange à 97 °C, puis on ajoute progressivement (en 20 à 30 minutes) 6 parties d'hydrate d'hydrazine 100 % et maintient ensuite à 100 °C pendant environ 12 heures.

On élimine une partie de l'eau par distillation jusqu'à l'obtention d'une pâte humide qu'on essore, puis lave à l'eau froide jusqu'à pH neutre et séche à l'étuve sous vide à 100-110 °C pendant 8 heures. On obtient ainsi un produit légérement coloré composé d'environ :

65 % de N,N′-bis(bromendimide)

17,5 % de N-amino bromendimide

et

17,5 % d'anhydride bromendique.

EXEMPLE 9 (comparatif)

Dans le même appareillage qu'à l'exemple 1, on dissout à 66 °C 619 parties d'anhydride tétrabromophtalique dans 1500 parties de méthanol. Après complète dissolution, on ajoute 34 parties d'hydrate d'hydrazine 100 %, puis on maintient à 67 °C pendant environ 8 heures.

Aprés refroidissement, filtration et lavage au méthanol, puis à l'eau, on obtient avec un rendement d'environ 75 % un produit jaune ne contenant pas d'anhydride tétrabromophtalique et constitué principalement par du N-amino tétrabromophtalimide avec un peu de N,N′-bis(tétrabromophtalimide) et d'autres produits non identifiés

EXEMPLE 10 (comparatif)

Dans le même appareillage qu'à l'exemple 1, on introduit 619 parties d'anhydride tétrabromophtalique dans 750 parties de diméthylformamide et 1250 parties de xylène, puis on porte le mélange à 100 °C. On obtient alors une solution jaune à laquelle on ajoute 34 parties d'hydrate d'hydrazine 100 %. On porte ensuite à 138 °C et maintient à cette température pendant environ 8 heures, tout en éliminant l'eau de formation par distillation azéotropique avec le xylène.

Après refroidissement, filtration, lavage à l'hexane et séchage sous vide, on obtient avec un rendement d'environ 78% un produit jaune ne contenant pas d'anhydride tétrabromophtalique et constitué principalement par du N,N′-bis(tétrabromophtalimide), avec un peu de N-amino tétrabromophtalimide et d'autres produits non identifiés.

EXEMPLE 11

L'efficacité des produits selon l'invention comme agents d'ignifugation des matières plastiques a été testée suivant le mode opératoire général suivant :

A l'aide d'un mélangeur du type planétaire ou "tonneau", on mélange dans les proportions indiquées dans les tableaux suivants la résine sous forme de poudre ou de granulés avec l'ignifugeant à tester et les additifs éventuels (trioxyde d'antimoine, paraffine). Aprés homogénéisation, on extrude le mélange au moyen d'une extrudeuse appropriée (type double-vis, mono-vis ou "Buss") équipée ou non d'un moyen de dégazage, en opérant à la température d'extrusion indiquée par le fournisseur de résine et liée à la température du fusion du polymère. Le compound obtenu, éventuellement granulé, est ensuite transformé en éprouvettes de mesure au moyen d'une presse à injecter opérant aux températures convenables. Ces éprouvettes sont ensuite soumises aux tests normalisés de résistance au feu UL94 en 3,2 mm et 1,6 mm (Norme NF T51072) et d'indice d'oxygène IO % (Norme NF T51071).

Le tableau A résume les résultats obtenus dans le cas du polybutylène téréphtalate (TMNO-ORGATER, granulés commercialisés par la Demanderesse).

Le tableau B rassemble les résultats obtenus avec le produit de l'exemple 1 et d'autres résines, à savoir :

- Polypropylène (PK-1060 P commercialisé par la Société HOECHST sous forme de poudre),
- Polyamide 12 (AMNO-RILSAN commercialisé par la Demanderesse sous forme de granulés),

- Résine ABS (UGIKRAL SF commercialisé par la Société CdF-Chimie sous forme de poudre).

TABLEAU A

| ESSAI N° | I temoin | II | III | IV | V | VI | VII (comparatifs) | VIII (comparatifs) |
|---|---|---|---|---|---|---|---|---|
| Ignifugeant de l'exemple n° | Néant | 1 | 4 | 5 | 6 | 7 | 9 | 10 |
| Composition (parties en poids) : | | | | | | | | |
| - Résine TMNO-ORGATER | 1000 | 790 | 3160 | 3449 | 3402 | 3290 | 1559 | 1559 |
| - Ignifugeant | 0 | 126 | 588 | 308 | 355 | 467 | 290 | 290 |
| - Trioxyde d'antimoine | 0 | 60 | 246 | 243 | 243 | 243 | 121 | 121 |
| - Paraffine | 0 | 15 | 60 | 60 | 60 | 60 | 30 | 30 |
| Résultats des tests : | | | | | | | | |
| - UL94 | NC | VO | VO | V2 | V2 | VO | VO | VO |
| - IO % | 22 | 32,6 | 31,3 | 25,1 | 23,7 | 27,6 | 30,1 | 31,7 |
| Couleur de l'éprouvette : | blanc | blanc-ivoire | blanc | légérement gris | blanc ivoire | blanc ivoire | jaune | jaune |

TABLEAU B

| RESINE | Composition (parties en poids) | | | Résultats des tests | | Couleur de l'éprouvette |
|---|---|---|---|---|---|---|
| | Résine | Ignifugeant de l'exemple 1 | Trioxide d'antimoine | UL94 | IO % | |
| PK 1060 P | 1000 (témoin) | 0 | 0 | NC | 18 | blanc |
| " | 522 | 359 | 119 | VO | 25 | blanc-ivoire |
| AMNO-RILSAN | 1000 (témoin) | 0 | 0 | V2 (en 3,2 mm) | 22 | légèrement jaune |
| " | 877 | 96 | 26 | VO | 29,4 | jaune pâle |
| UGIKRAL SF | 1000 (témoin) | 0 | 0 | NC | 18 | beige |
| " | 766 | 150 | 50 | VO (en 3,2 mm) | 25,6 | beige clair |

## Revendications

1. Procédé de préparation d'imides halogénés par condensation d'hydrazine et d'anhydride d'acide dicarboxylique halogéne de formule

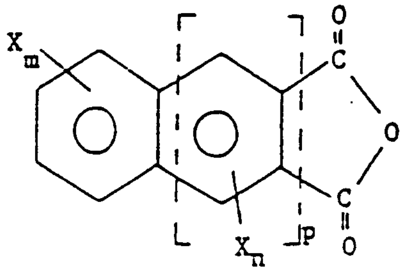 ou 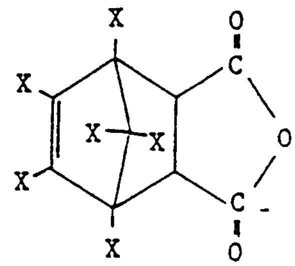

où X représente un atome de brome ou de chlore, m est un nombre entier allant de 2 à 4, n et p sont des nombres entiers allant de 0 à 2, ou un mélange de tels anhydrides, caractérisé en ce que la réaction est effectuée en milieu aqueux, à une température allant de 40 à 225 °C, avec un rapport molaire anhydride/hydrazine sensiblement égal à 2.

2. Procédé selon la revendication 1, dans lequel on opère sous pression à une température d'au moins 140 °C.

3. Procédé selon la revendication 1 ou 2, dans lequel on part d'une solution ou dispersion aqueuse d'anhydride ayant un pH inférieur à 4.

4. Procédé selon la revendication 1, dans lequel on utilise l'anhydride tétrabromophtalique, l'anhydride tétraclorophtalique, l'anhydride chlorendique, l'anhydride bromendique ou en mélange de ces anhydride.

5. Procédé selon la revendication 1 à 4, dans lequel l'hydrazine est utilisée sous forme d'hydrate ou de sel d'hydrazinium.

6. Compositions ignifugeantes à base de bis-imide d'acide dicarboxylique halogéné sensiblement non coloré, pouvant être obtenues selon l'une des revendications 1 à 5, résultant de la condensation d'hydrazine et d'anhydride d'acide dicarboxylique halogéné de formule

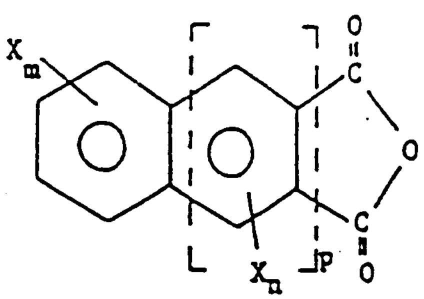 ou 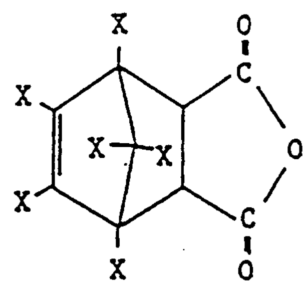

où X représente un atome de brome ou de chlore, m est un nombre entier allant de 2 à 4, n et p sont des nombres entiers allant de 0 à 2, ou un mélange de tels anhydrides, caractérisées en ce qu'elles contiennent en plus du bis-imide précité également jusqu'à 80 % en poids d'un mélange sensiblement équimolaire du N-amino-imide et de l'anhydride dudit acide dicarboxylique halogéné.

7. Compositions selon la revendition 6, constituées par du N,N'-bis(tétrabromophtalimide), du N-amino tétrabromophtalimide et de l'anhydride tétrabromophtalique.

8. Application des produits obtenus selon le procédé de l'une des revendications 1 à 5 ou des compositions selon la revendication 6 ou 7, à l'ignifugation des matières plastiques.

9. Matières plastiques ignifugées au moyen d'un produit obtenu selon le procédé de l'une des revendications 1 à 5 ou d'une composition selon la revendication 6 ou 7.

## Claims

1. Process for the preparation of halo imides by condensation of hydrazine and a halo dicarboxylic acid anhydride of formula

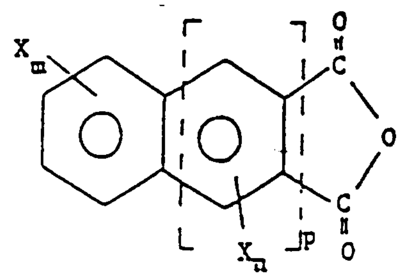

or

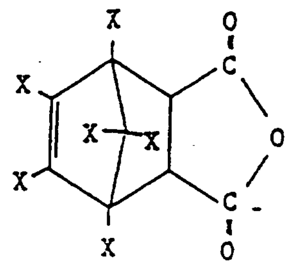

where X represents a bromine or chlorine atom, m is an integer ranging from 2 to 4 and n and p are integers ranging from 0 to 2, or a mixture of such anhydrides, characterized in that the reaction is carried out in an aqueous medium, at a temperature ranging from 40 to 225 ° C, with an anhydride/hydrazine molar ratio substantially equal to 2.

2. Process according to Claim 1, in which the process is performed at a temperature of at least 140 ° C.

3. Process according to Claim 1 or 2, in which an aqueous anhydride solution or dispersion having a pH lower than 4 is used as starting material.

4. Process according to Claim 1, in which tetrabromophthalic anhydride, tetrachlorophthalic anhydride, chlorendic anhydride, bromendic anhydride or a mixture of these anhydrides is used.

5. Process according to Claims 1 to 4, in which hydrazine is used in the hydrate or hydrazinium salt form.

6. Flame retardant compositions based on substantially colourless halo dicarboxylic acid bisimide, which may be obtained according to one of Claims 1 to 5, resulting from the condensation of hydrazine and halo dicarboxylic acid anhydride of formula

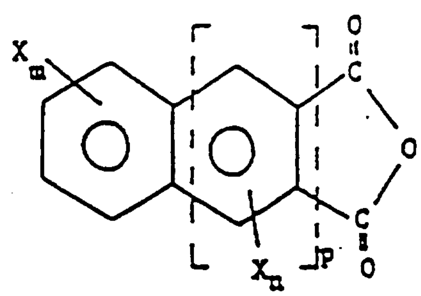

or

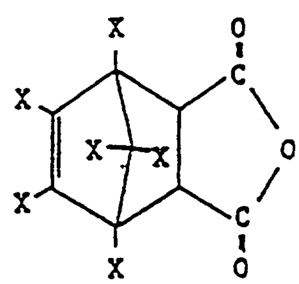

where X represents a bromine or chlorine atom, m is an integer ranging from 2 to 4 and n and p are integers ranging from 0 to 2, or a mixture of such anhydrides, characterized in that they contain, in addition to the abovementioned bisimide, also up to 80 % by weight of a substantially equimolar mixture of the N-aminoimide and the anhydride of the said halo dicarboxylic acid.

7. Compositions according to Claim 6, consisting of N,N'-bis(tetrabromophthalimide), N-amino-tetrabromophthalimide and tetrabromophthalic anhydride.

8. Application of the products obtained according to the process of one of Claims 1 to 5 or of the compositions according to Claim 6 or 7, to the flame retarding treatment of plastics.

**9.** Plastics which are given a flame retarding treatment using a product obtained according to the process of one of Claims 1 to 5 or using a composition according to Claim 6 or 7.

**Patentansprüche**

**1.** Verfahren zur Herstellung von halogenierten Imiden durch Kondensation von Hydrazin mit einem halogenierten Dicarbonsäureanhydrid der Formel

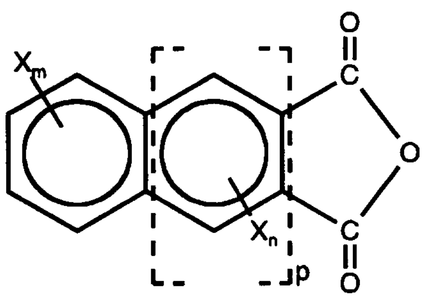

oder

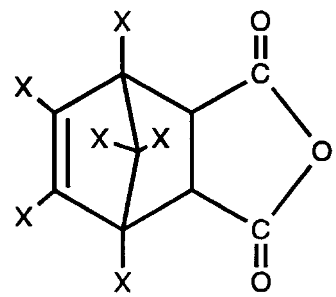

in der X ein Brom- oder Chloratom darstellt, m eine ganze Zahl zwischen 2 und 4 ist und n und p eine ganze Zahl zwischen 0 und 2 darstellt,
oder eine Mischung solcher Anhydride, dadurch gekennzeichnet, daß die Reaktion in wäßrigem Milieu bei einer Temperatur zwischen 40 und 225 °C mit einem Anhydrid/Hydrazin-Molvehältnis von ungefähr gleich 2 durchgeführt wird.

**2.** Verfahren nach Anspruch 1, bei dem man unter Druck bei einer Temperatur von mindestens 140 °C verfährt.

**3.** Verfahren nach Anspruch 1 oder 2, bei dem man von einer wäßrigen Lösung oder Dispersion des Anhydrids mit einem pH-Wert Kleiner als 4 ausgeht.

**4.** Verfahren nach Anspruch 1, bei dem man Tetrabromphthalsäureanhydrid, Tetrachlorphthalsäureanhydrid, Chlorendic-anhydrid, Bromendicanhydrid oder eine Mischung dieser Anhydride verwendet.

**5.** Verfahren nach Anspruch 1 bis 4, bei dem man das Hydrazin als Hydratverbindung oder Hydraziniumsalz eingesetzt wird.

**6.** Flammhemmende Mischungen auf Basis von praktisch farblosem, halogeniertem Dicarbonsäurebisimid, die nach einem der Ansprüche 1 bis 5 durch Kondensation von Hydrazin mit einem halogenierten Dicarbonsäureanhydrid der Formel

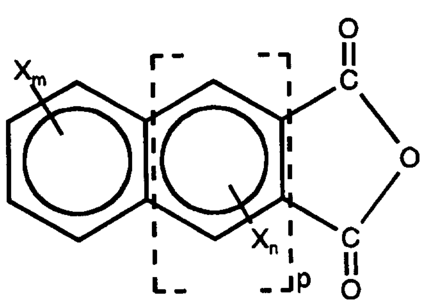

oder

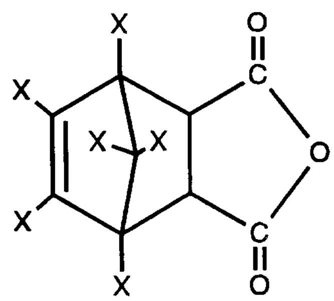

in der X ein Brom- oder Chloratom, m eine ganze Zahl zwischen 2 und 4 ist, n und p eine ganze Zahl zwischen 0 und 2 darstellen,
oder eine Mischung solcher Anhydride erhalten werden können, dadurch gekennzeichnet, daß sie zusätzlich zu dem vorgenannten Bisimid gleichzeitig bis zu 80 Gew.% einer ungefähr equimolaren

Mischung von N-Aminoimid und dem Anhydrid der genannten halogenierten Dicarbonsäure enthält.

**7.** Mischung nach Anspruch 6, dargestellt aus N,N'-bis(tetrabromphthalimid), N-Amino-tetrabromphthalimid und Tetrabromphthalsäureanhydrid.

**8.** Verwendung der nach einem Verfahren der Ansprüche 1 bis 5 oder der Mischungen nach Anspruch 6 oder 7 erhaltenen Produkte zur Flammhemmung von Kunststoffen.

**9.** Kunststoffe, die mit Hilfe eines nach dem Verfahren eines der Ansprüche 1 bis 5 erhaltenen Produktes oder einer Zusammensetzung nach Anspruch 6 oder 7 flammhemmend gemacht wurden.